# EUROPEAN PATENT APPLICATION

(11) **EP 1 854 424 A1**
(43) Date of publication of application: **14.11.2007**
(21) Application number: 07009321.6
(22) Date of filing: 09.05.2007
(51) Int. Cl.: A61B 18/14

(54) **Vessel sealing instrument with optimized power density**

(30) Priority: 10.05.2006 US 431466
(71) Applicant: Covidien AG, 8212 Neuhausen am Rheinfall (CH)
(72) Inventor: Couture, Gary M., Longmont CO 80501 (US); Sharp, Robert, Boulder CO 80304 (US); Unger, Jeff, Superior, CO 80027 (US)
(74) Representative: HOFFMANN EITLE

(57) **Abstract**

An electrode assembly for use with an instrument for sealing and cutting vessels and/or tissue is provided. The assembly includes a pair of opposing first and second jaw members. Each jaw member includes an insulator and at least one electrically conductive tissue sealing surface extending along a length thereof, each tissue sealing surface being adapted to connect to a source of electrosurgical energy to effect a seal. At least one electrically conductive cutting element is disposed within the insulator of the first jaw member and an insulative material is disposed upon the at least one electrically conductive sealing surface.

## Description

### BACKGROUND

### Technical Field

The present disclosure relates to a forceps used for both endoscopic and open surgical procedures that includes an electrode assembly that allows a user to selectively seal and/or cut tissue. More particularly, the present disclosure relates to a forceps that applies a unique combination of mechanical clamping pressure and electrosurgical energy to effectively seal and sever tissue between sealed tissue areas.

### Background of Related Art

Open or endoscopic electrosurgical forceps utilize both mechanical clamping action and electrical energy to effect hemostasis. The electrode of each opposing jaw member is charged to a different electric potential such that when the jaw members grasp tissue, electrical energy can be selectively transferred through the tissue. A surgeon can either cauterize, coagulate/desiccate and/or simply reduce or slow bleeding, by controlling the intensity, frequency and duration of the electrosurgical energy applied between the electrodes and through the tissue.

Certain surgical procedures require more than simply cauterizing tissue and rely on the combination of clamping pressure, electrosurgical energy and gap distance (i.e., distance between opposing jaw members when closed about tissue) to "seal" tissue, vessels and certain vascular bundles. More particularly, vessel sealing or tissue sealing is a recently-developed technology that utilizes a unique combination of radiofrequency energy, clamping pressure and precise control of gap distance to effectively seal or fuse tissue between two opposing jaw members or sealing plates. Vessel or tissue sealing is more than "cauterization", which involves the use of heat to destroy tissue (also called "diathermy" or "electrodiathermy"). Vessel sealing is also more than "coagulation", which is the process of desiccating tissue wherein the tissue cells are ruptured and dried. "Vessel sealing" is defined as the process of liquefying the collagen, elastin and ground substances in the tissue so that the tissue reforms into a fused mass with significantly-reduced demarcation between the opposing tissue structures.

To effectively seal tissue or vessels, especially thick tissue and large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel or tissue being sealed. Accurate application of pressure is important for several reasons: to oppose the walls of the vessel; to reduce the tissue impedance to a low enough value that allows enough electrosurgical energy through the tissue; to overcome the forces of expansion during tissue heating; and to contribute to the end tissue thickness, which is an indication of a good seal. It has been determined that a typical fused vessel wall is optimum between about 0.001 and about 0.006 inches. Below this range, the seal may shred or tear and above this range the tissue may not be properly or effectively sealed.

With respect to smaller vessels, the pressure applied becomes less relevant and the gap distance between the electrically conductive surfaces becomes more significant for effective sealing. In other words, the chances of the two electrically conductive surfaces touching during activation increases as the tissue thickness and the vessels become smaller.

Typically, and particularly with respect to endoscopic electrosurgical procedures, once a vessel is sealed, the surgeon has to remove the sealing instrument from the operative site, substitute a new instrument through the cannula and accurately sever the vessel along the newly formed tissue seal. As can be appreciated, this additional step may be both time-consuming (particularly when sealing a significant number of vessels) and may contribute to imprecise separation of the tissue along the sealing line due to the misalignment or misplacement of the severing instrument along the center of the tissue seal.

Several attempts have been made to design an instrument that incorporates a knife or blade member, which effectively severs the tissue after forming a tissue seal.

Sealing and electrical cutting on the same instrument is a recently developed technology that provides different advantages over mechanically cutting tissue. However, electrical cutting of tissue has proven difficult for manufacturing due to the dimensions between electrodes being relatively small. The electrodes may produce heat formation and electrical charging during the seal cycle that detrimentally affects the cut performance. This may manifest itself by damaging tissue within the cut zone and minimizing hydration by forcing conductive fluids from the cut area.

### SUMMARY

Accordingly, the present disclosure is directed to an electrode assembly for use with an instrument for sealing and cutting vessels and/or tissue. In one embodiment the assembly includes a pair of opposing first and second jaw members at least one of which being movable relative to the other from a first position wherein the jaw members are disposed in spaced relation relative to one another to a second position wherein the jaw members cooperate to grasp tissue therebetween.

Each jaw member includes an insulator and at least one electrically conductive tissue sealing surface extending along a length thereof, each tissue sealing surface being adapted to connect to a source of electrosurgical energy such that the tissue sealing surfaces are capable of conducting electrosurgical energy through tissue held therebetween to effect a seal.

The first jaw member includes at least one electrically conductive cutting element disposed within the insulator of the first jaw member, the electrically conductive cutting element disposed in general vertical registration to the insulator on the second jaw member defining at least one cutting zone between the at least one electrically conductive tissue sealing surfaces and the cutting element.

An insulative material is included that is disposed between at least one of the electrically conductive tissue sealing surfaces of the first jaw member between at least one electrically conductive tissue sealing surface of the first jaw member and the cutting element, the insulative material configured to reduce the exposed surface area of at least one electrically conductive tissue sealing surface of the first jaw member to optimize the power density for at least one of sealing and cutting tissue.

In another embodiment of the present disclosure a method of focusing energy to a specific area within tissue for use with an instrument for sealing and cutting vessels and/or tissue is provided. The method includes providing a pair of opposing jaw members each having an insulation and a pair of electrically conductive tissue sealing surfaces at least one of which being movable relative to the other from a first position wherein the jaw members are disposed in spaced relation relative to one another to a second position wherein the jaw members cooperate to grasp tissue therebetween. Each jaw member includes at least one electrically conductive tissue sealing surface extending along a length thereof, the jaw members adapted to connect to a source of electrosurgical energy such that the electrically conductive tissue sealing surfaces are capable of conducting electrosurgical energy through tissue held therebetween to effect a seal. The at least one electrically conductive tissue sealing surface having an exposed surface area and the first jaw member includes a cutting element disposed between electrically conductive tissue sealing surfaces. The method further includes positioning the opposing first and second jaw members about tissue and disposing an insulative material upon at least one electrically conductive tissue sealing surface of the first jaw member, the insulative material configured to reduce the exposed surface area of the at least one electrically conductive tissue sealing surface of the first jaw member. The method also includes adjusting the position of the insulative material on the electrically conductive tissue sealing surfaces to focus electrosurgical energy to a specific area between the cutting element and the electrically conductive tissue sealing surfaces of the jaw member.

### BRIEF DESCRIPTION OF THE DRAWINGS

Various embodiments of the subject instrument are described herein with reference to the drawings wherein:
FIG. 1A shows a right, perspective view of an endoscopic bipolar forceps having a housing, a shaft and a pair of jaw members affixed to a distal end thereof, the jaw members including an electrode assembly disposed therebetween;
FIG. 1 B shows a left, perspective view of an open bipolar forceps showing a pair of first and second shafts each having a jaw member affixed to a distal end thereof with an electrode assembly disposed therebetween;
FIG. 2 shows a cross-sectional view of a vessel sealing instrument showing one embodiment of a cut-zone configuration having an optimized power density;
FIG. 3 shows an enlarged cross-sectional view of the vessel sealing instrument showing a cut-zone configuration having an optimized power density;
FIG. 4 shows a cross-sectional view of the vessel sealing instrument showing a vessel held between the jaw members; and
FIG. 5 shows an alternate embodiment of the vessel sealing instrument having additional insulative material.

### DETAILED DESCRIPTION

For the purposes herein, vessel/tissue cutting or vessel/tissue division is believed to occur when heating of the vessel/tissue leads to expansion of intracellular and/or extra-cellular fluid, which may be accompanied by cellular vaporization, desiccation, fragmentation, collapse and/or shrinkage along a so-called "cut zone" in the vessel/tissue. By focusing the electrosurgical energy and heating in the cut zone, the cellular reactions are localized creating a fissure. Localization is achieved by regulating the vessel/tissue condition and energy delivery, which may be controlled by utilizing one or more of the various geometrical electrode and insulator configurations described herein. The cut process may also be controlled by utilizing a generator and feedback algorithm (and one or more of the hereindescribed geometrical configurations of the electrode and insulator assemblies), which increases the localization and maximizes the so-called "cutting effect".

For example, the below-described factors may contribute and/or enhance vessel/tissue division using electrosurgical energy. Each of the factors described below may be employed individually or in any combination to achieve a desired cutting effect. For the purposes herein the term "cut effect" or "cutting effect" refers to the actual division of tissue by one or more of the electrical or electro-mechanical methods or mechanisms described below. The term "cutting zone" or "cut zone" refers to the region of vessel/tissue where cutting will take place. The term "cutting process" refers to steps that are implemented before, during and/or after vessel/tissue division that tend to influence the vessel/tissue as part of achieving the cut effect.

For the purposes herein the terms "tissue" and "vessel" may be used interchangeably since it is believed that the present disclosure may be employed to seal and cut tissue or seal and cut vessels utilizing the same inventive principles described herein.

It is believed that the following factors either alone or in combination, play an important role in dividing tissue:
- Localizing or focusing electrosurgical energy in the cut zone during the cutting process while minimizing energy effects to surrounding tissues;
- Focusing the power density in the cut zone during the cutting process;
- Creating an area of increased temperature in the cut zone during the cutting process (e.g., heating that occurs within the tissue or heating the tissue directly with a heat source);
- Pulsing the energy delivery to influence the tissue in or around the cut zone. "Pulsing" involves as a combination of an "on" time and "off" time during which the energy is applied and then removed repeatedly at any number of intervals for any amount of time. The pulse "on" and "off' time may vary between pulses. The pulse "on" typically refers to a state of higher power delivery and pulse "off' typically refers to a state of lower power delivery;
- Spiking the energy delivery creates a momentary condition of high energy application with an intent to influence the tissue in or around the cut zone during the cut process. The momentary condition may be varied to create periods of high energy application;
- Conditioning the tissue before or during the cutting process to create more favorable tissue conditions for cutting. This includes tissue preheating before the cutting processes and tissue rehydration during the cutting process;
- Controlling the tissue volume in or around the cut zone to create more favorable conditions for tissue cutting;
- Controlling energy and power delivery to allow vaporization to enhance and or contribute to the cutting process. For example, controlling the energy delivery to vaporize both intracellular and/or extracellular fluids and/or other cellular materials and foreign fluids within the cut zone;
- Fragmenting the tissue or cellular material during the cutting process to enhance tissue division in the cut zone;
- Melting or collapsing the tissue or cellular material during the cutting process to enhance tissue division in the cut zone. For example, melting the tissue to create internal stress within the tissue to induce tissue tearing;
- Controlling tissue temperature, arcing, power density and/or current density during the cutting process to enhance tissue division in the cut zone;
- Applying various mechanical elements to the tissue such as pressure, tension and/or stress (either internally or externally) to enhance the cutting process; and
- Utilizing various other tissue treatments before or during the cutting process to enhance tissue cutting, e.g., tissue sealing, cauterization and/or coagulation.

Many of the electrode assemblies described herein employ one or more of the above-identified factors for enhancing tissue division. For example, many of the electrode assemblies described herein utilize various geometrical configurations of electrodes, cutting elements, insulators, partially conductive materials and semiconductors to produce or enhance the cutting effect. In addition, by controlling or regulating the electrosurgical energy from the generator in any of the ways described above, tissue cutting may be initiated, enhanced or facilitated within the tissue cutting zone. For example, it is believed that the geometrical configuration of the electrodes and insulators may be configured to produce a so-called "cut effect", which may be directly related to the amount of vaporization or fragmentation at a point in the tissue or the power density, temperature density and/or mechanical stress applied to a point in the tissue. The geometry of the electrodes may be configured such that the surface area ratios between the electrical poles focus electrical energy at the tissue. Moreover, the geometrical configurations of the electrodes and insulators may be designed such that they act like electrical sinks or insulators to influence the heat effect within and around the tissue during the sealing or cutting processes.

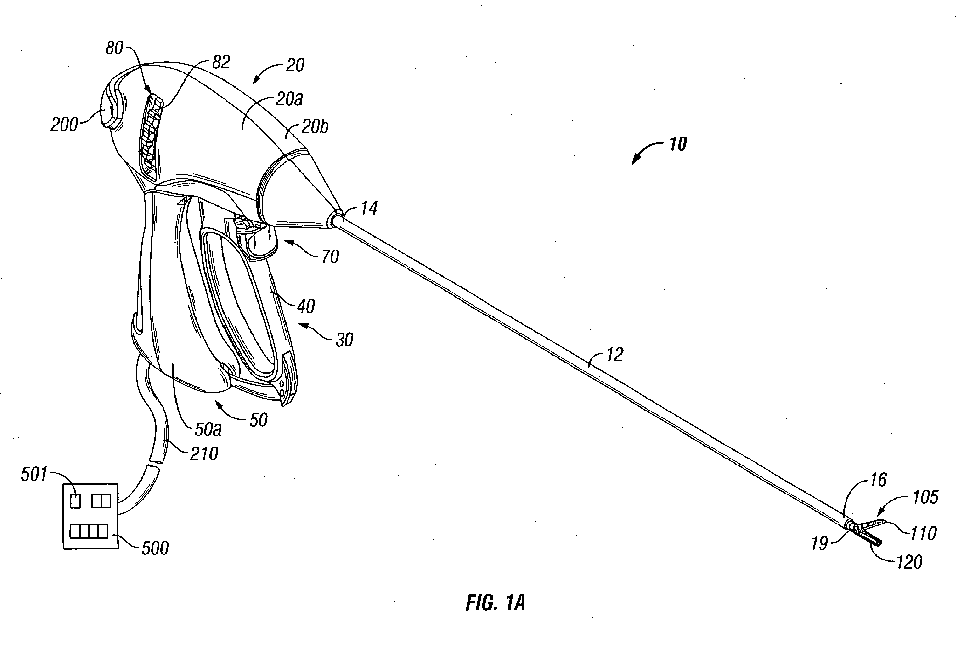

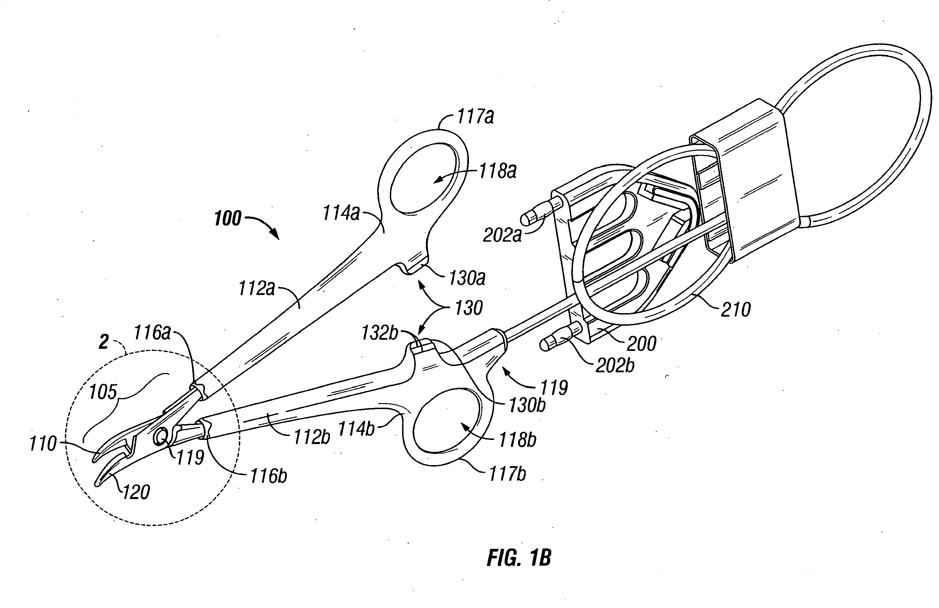

Referring now to the various figures, FIG. 1A depicts a bipolar forceps 10 for use in connection with endoscopic surgical procedures and Fig. 1 B depicts an open forceps 100 contemplated for use in connection with traditional open surgical procedures. For the purposes herein, either an endoscopic instrument or an open instrument may be utilized with the electrode assembly described herein. Obviously, different electrical and mechanical connections and considerations apply to each particular type of instrument; however, the novel aspects with respect to the electrode assembly and its operating characteristics remain generally consistent with respect to both the open or endoscopic designs.

Fig. 1A shows a bipolar forceps 10 for use with various endoscopic surgical procedures and generally includes a housing 20, a handle assembly 30, a rotating assembly 80, a switch assembly 70 and an electrode assembly 105 having opposing jaw members 110 and 120 which mutually cooperate to grasp, seal and divide tubular vessels and vascular tissue. More particularly, forceps 10 includes a shaft 12 which has a distal end 16 dimensioned to mechanically engage the electrode assembly 105 and a proximal end 14 which mechanically engages the housing 20. The shaft 12 may include one or more known mechanically engaging components which are designed to securely receive and engage the electrode assembly 105 such that the jaw members 110 and 120 are pivotable relative to one another to engage and grasp tissue therebetween.

The proximal end 14 of shaft 12 mechanically engages the rotating assembly 80 (not shown) to facilitate rotation of the electrode assembly 105. In the drawings and in the descriptions which follow, the term "proximal", as is traditional, will refer to the end of the forceps 10 which is closer to the user, while the term "distal" will refer to the end which is further from the user.

Handle assembly 30 includes a fixed handle 50 and a movable handle 40. Fixed handle 50 is integrally associated with housing 20 and handle 40 is movable relative to fixed handle 50 to actuate the opposing jaw members 110 and 120 of the electrode assembly 105 as explained in more detail below. Movable handle 40 and switch assembly 70 are of unitary construction and are operatively connected to the housing 20 and the fixed handle 50 during the assembly process. Housing 20 is constructed from two components halves 20a and 20b which are assembled about the proximal end of shaft 12 during assembly. Switch assembly is configured to selectively provide electrical energy to the electrode assembly 105.

As mentioned above, electrode assembly 105 is attached to the distal end 16 of shaft 12 and includes the opposing jaw members 110 and 120. Movable handle 40 of handle assembly 30 imparts movement of the jaw members 110 and 120 from an open position wherein the jaw members 110 and 120 are disposed in spaced relation relative to one another, to a clamping or closed position wherein the jaw members 110 and 120 cooperate to grasp tissue therebetween.

Referring now to Fig. 1 B, an open forceps 100 includes a pair of elongated shaft portions 112a and 112b each having a proximal end 114a and 114b, respectively, and a distal end 116a and 116b, respectively. The forceps 100 includes jaw members 120 and 110 which attach to distal ends 116a and 116b of shafts 112a and 112b, respectively. The jaw members 110 and 120 are connected about pivot pin 119 which allows the jaw members 110 and 120 to pivot relative to one another from the first to second positions for treating tissue. The electrode assembly 105 is connected to opposing jaw members 110 and 120 and may include electrical connections through or around the pivot pin 119.

Each shaft 112a and 112b includes a handle 117a and 117b disposed at the proximal end 114a and 114b thereof which each define a finger hole 118a and 118b, respectively, therethrough for receiving a finger of the user. As can be appreciated, finger holes 118a and 118b facilitate movement of the shafts 112a and 112b relative to one another which, in turn, pivot the jaw members 110 and 120 from the open position wherein the jaw members 110 and 120 are disposed in spaced relation relative to one another to the clamping or closed position wherein the jaw members 110 and 120 cooperate to grasp tissue therebetween. A ratchet 130 is included for selectively locking the jaw members 110 and 120 relative to one another at various positions during pivoting.

More particularly, the ratchet 130 includes a first mechanical interface 130a associated with shaft 112a and a second mating mechanical interface associated with shaft 112b. Each position associated with the cooperating ratchet interfaces 130a and 130b holds a specific, i.e., constant, strain energy in the shaft members 112a and 112b which, in turn, transmits a specific closing force to the jaw members 110 and 120. It is envisioned that the ratchet 130 may include graduations or other visual markings which enable the user to easily and quickly ascertain and control the amount of closure force desired between the jaw members 110 and 120.

As best seen in Fig. 1B, forceps 100 also includes an electrical interface or plug 200 which connects the forceps 100 to a source of electrosurgical energy, e.g., an electrosurgical generator (not shown). Plug 200 includes at least two prong members 202a and 202b which are dimensioned to mechanically and electrically connect the forceps 100 to the electrosurgical generator 500 (See Fig. 1 A). An electrical cable 210 extends from the plug 200 and securely connects the cable 210 to the forceps 100. Cable 210 is internally divided within the shaft 112b to transmit electrosurgical energy through various electrical feed paths to the electrode assembly 105.

One of the shafts, e.g., 112b, includes a proximal shaft connector/flange 119 which is designed to connect the forceps 100 to a source of electrosurgical energy such as an electrosurgical generator 500. More particularly, flange 119 mechanically secures electrosurgical cable 210 to the forceps 100 such that the user may selectively apply electrosurgical energy as needed.

It is envisioned that the cutting element may be substantially dull and only capable of cutting tissue through electrosurgical activation. Moreover, the cutting element may be disposed within the insulator of the first or second jaw member. As mentioned hereinbefore the potential of the cutting element and the electrically conductive tissue sealing surfaces may be altered depending upon a particular desired surgical effect.

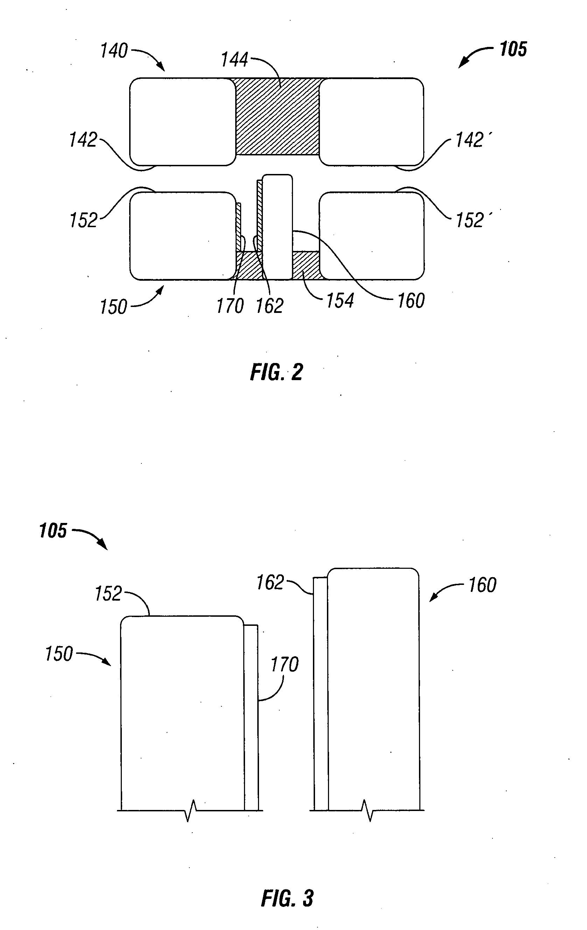

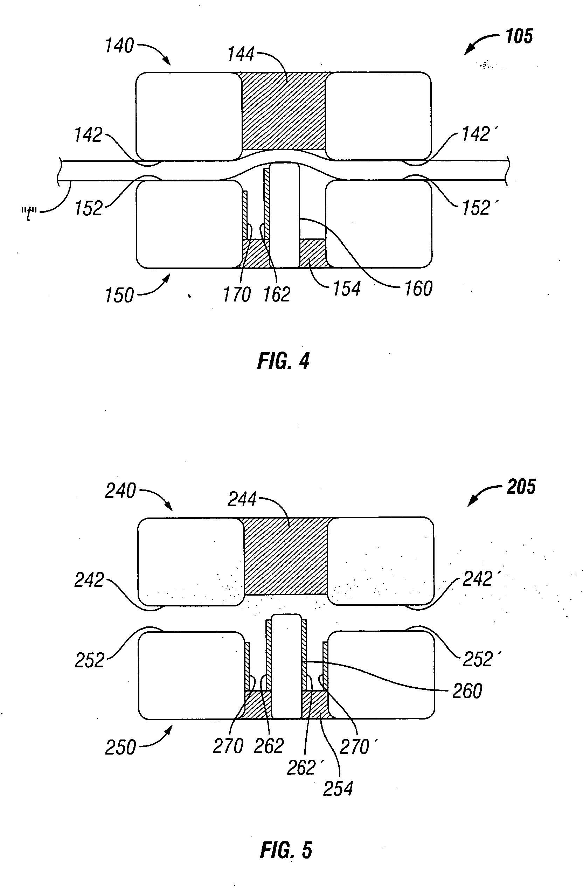

FIGS. 2-4 show an electrode assembly 105 for use with an instrument for sealing and cutting vessels and/or tissue. Electrode assembly 105 includes a pair of opposing first 140 and second 150 jaw members at least one of which is movable relative to the other from a first position wherein the jaw members 140, 150 are disposed in spaced relation relative to one another to a second position wherein the jaw members cooperate to grasp tissue "t" therebetween (FIG. 4). Each jaw member 140, 150 includes at least one electrically conductive tissue sealing surface or electrode 142, 142', 152, 152' extending along a length thereof, each tissue sealing surface 142, 142', 152, 152' is adapted to connect to a source of electrosurgical energy such that the electrically conductive tissue sealing surfaces 142, 142', 152, 152' are capable of conducting electrosurgical energy through tissue "t" held therebetween to effect a seal. The electrically conductive tissue sealing surfaces 142, 142', 152, 152' have an exposed surface area.

Electrode assembly 105 also includes at least one electrically conductive cutting element 160 disposed within the insulator 144(not shown), 154 of the first or second jaw member 140, 150. Cutting element 160 is disposed in general vertical registration to jaw member 140 and extends outwardly from the insulator 154 of jaw member 150. Cutting element 160 defines at least one cutting zone between the electrically conductive tissue sealing surfaces 142, 142', 152, 152' and cutting element 160. Cutting element 160 may include an insulative material 162 disposed thereupon. Insulative material 162 is configured to work in conjunction with other factors described below to focus the energy intensity (or direction of the power density) to facilitate tissue sealing and/or cutting.

Electrode assembly 105 further includes an insulator or insulative material 170 disposed upon electrically conductive tissue sealing surface 152. The insulative material 170 is configured to reduce the exposed surface area of electrically conductive tissue sealing surface 152, which localizes current density or energy intensity. The other electrically conductive surfaces 142, 142', and 152' may also include an insulated material disposed thereon. Manipulating the amount of insulative material on the electrically conductive tissue sealing surfaces 142, 142', 152, 152' or on the electrically conductive cutting element 160 is believed to provide optimal power density for sealing and/or cutting. The insulative material may include, but is not limited to, glass, ceramic, polymeric and other suitable materials.

In order to effect tissue sealing, using the configuration shown in FIGS. 2-4, electrically conductive tissue sealing surfaces 142, 142' should maintain a positive polarity while sealing surfaces 152, 152' should maintain a negative polarity. This configuration, in conjunction with a neutral electrically conductive cutting element 160, is desirable to effect tissue sealing. However, numerous alternative configurations are also within the scope of the present disclosure.

In order to effect tissue cutting, using the configuration shown in FIGS. 2-4, electrically conductive tissue sealing surfaces 142, 142', 152 and 152' may have a negative polarity while electrically conductive cutting element 160 maintains a positive polarity. However, as mentioned above, this configuration is merely indicative of one possible tissue cutting configuration. For example, the polarities of the sealing surfaces 142, 142', 152, 152' and cutting element 160 could be reversed to yield a similar result.

Electrode assembly 105 may be configured in a variety of different arrangements. In one embodiment, the exposed surface area of electrically conductive tissue sealing surface 152 and electrically conductive cutting element 160 is substantially the same. Alternatively, the exposed area of electrically conductive tissue sealing surface 152 may be greater than the exposed area of electrically conductive cutting element 160. The electrode (in FIG. 2 either sealing surface 152 or cutting element 160) with the smallest ratio of exposed surface area will have the highest concentration of current density or power. By manipulating this ratio additional control over the sealing and cutting processes is achieved.

In operation, electrode assembly 105 is used to focus energy/power density to a specific area within tissue by controlling the exposed surface area on one or all of the electrodes 142, 142', 152, 152', the electrically conductive cutting element 160 or both the electrodes 142, 142', 152, 152' and cutting electrode 160. By varying the ratio of the exposed surface area of electrodes 142, 142', 152, 152' and the cutting element 160, energy may be focused within the cut zone. For instance, if the surface area of each electrode 142, 142', 152, 152' or cutting element 160 is substantially equivalent (i.e., a 1:1 ratio), the power density created will be centered in the area between any 2 electrodes. Any variance in this ratio will shift the power density towards the electrode with the smallest surface area. Moreover, the energy may be focused where tissue has a smaller cross-sectional area thus facilitating tissue division. The distance between the first 140 and second 150 jaw members and the position of the insulative material 162, 170 may be manipulated to focus energy to a specific area depending upon a particular tissue type or surgical purpose.

Referring now to FIG. 5, an example of an alternate embodiment of electrode assembly 205 is shown. In this configuration additional insulative material 262' and 270' is provided that minimizes the exposed surface areas of electrodes 252 and 252' and cutting element 260 thus increasing the power density within the cutting zone.

As can be appreciated, the various geometrical configurations and electrical arrangements of the aforementioned electrode assemblies allow the surgeon to initially activate the two opposing electrically conductive tissue contacting surfaces and seal the tissue and, subsequently, selectively and independently activate the cutting element and one or more tissue contacting surfaces to cut the tissue utilizing the various above-described and shown electrode assembly configurations. Hence, the tissue is initially sealed and thereafter cut without re-grasping the tissue.

However, the cutting element and one or more tissue contacting surfaces may also be activated to simply cut tissue/vessels without initially sealing. For example, the jaw members may be positioned about tissue and the cutting element may be selectively activated to separate or simply coagulate tissue. This type of alternative embodiment may be particularly useful during certain endoscopic procedures wherein an electrosurgical pencil is typically introduced to coagulate and/or dissect tissue during the operating procedure.

A switch 70 may be employed to allow the surgeon to selectively activate one or more tissue contacting surfaces or the cutting element independently of one another. As can be appreciated, this allows the surgeon to initially seal tissue and then activate the cutting element by simply activating the switch. Rocker switches, toggle switches, flip switches, dials, etc. are types of switches that can be commonly employed to accomplish this purpose.

These switches can be placed anywhere on the instrument or may be configured as a remote switch, .e.g., handswitch or footswitch. The switch may also cooperate with a smart sensor 501 (or smart circuit, computer, feedback loop, etc.) that automatically triggers the switch to change between the "sealing" mode and the "cutting" mode upon the satisfaction of a particular parameter (Fig. 1A). For example, the smart sensor may include a feedback loop that indicates when a tissue seal is complete based upon one or more of the following parameters: tissue temperature, tissue impedance at the seal, change in impedance of the tissue over time and/or changes in the power or current applied to the tissue over time. An audible or visual feedback monitor may be employed to convey information to the surgeon regarding the overall seal quality or the completion of an effective tissue seal. A separate lead may be connected between the smart sensor and the generator for visual and/or audible feedback purposes.

The generator 500 delivers energy to the tissue in a pulse-like waveform. It has been determined that delivering the energy in pulses increases the amount of sealing energy which can be effectively delivered to the tissue and reduces unwanted tissue effects such as charring. Moreover, the feedback loop of the smart sensor can be configured to automatically measure various tissue parameters during sealing (i.e., tissue temperature, tissue impedance, current through the tissue) and automatically adjust the energy intensity and number of pulses as needed to reduce various tissue effects such as charring and thermal spread.

It has also been determined that RF pulsing may be used to more effectively cut tissue. For example, an initial pulse from the cutting element through the tissue (or the tissue contacting surfaces through the tissue) may be delivered to provide feedback to the smart sensor for selection of the ideal number of subsequent pulses and subsequent pulse intensity to effectively and consistently cut the amount or type of tissue with minimal effect on the tissue seal. If the energy is not pulsed, the tissue may not initially cut but desiccate since tissue impedance remains high during the initial stages of cutting. By providing the energy in short, high energy pulses, it has been found that the tissue is more likely to cut.

Alternatively, a switch may be configured to activate based upon a desired cutting parameter and/or after an effective seal is created or has been verified. For example, after effectively sealing the tissue, the cutting element may be automatically activated based upon a desired end tissue thickness at the seal.

As mentioned in many of the above embodiments, upon compression of the tissue, the cutting element may act as a stop member and create a gap "G" between the opposing conductive tissue contacting surfaces. Particularly with respect to vessel sealing, the gap distance is in the range of about 0.001 to about 0.006 inches. As mentioned above, controlling both the gap distance "G" and clamping pressure between conductive surfaces are two important mechanical parameters that need to be properly controlled to assure a consistent and effective tissue seal. The surgeon activates the generator to transmit electrosurgical energy to the tissue contacting surfaces and through the tissue to effect a seal. As a result of the unique combination of the clamping pressure, gap distance "G" and electrosurgical energy, the tissue collagen melts into a fused mass with limited demarcation between opposing vessel walls.

Once sealed, the surgeon activates the cutting element to cut the tissue. As mentioned above, the surgeon does not necessarily need to re-grasp the tissue to cut, i.e., the cutting element is already positioned proximate the ideal, center cutting line of the seal. During the cutting phase, highly concentrated electrosurgical energy travels from the cutting element through the tissue to cut the tissue into two distinct halves. As mentioned above, the number of pulses required to effectively cut the tissue and the intensity of the cutting energy may be determined by measuring the seal thickness and/or tissue impedance and/or based upon an initial calibrating energy pulse which measures similar parameters. A smart sensor (not shown) or feedback loop may be employed for this purpose.

As can be appreciated, the forceps may be configured to automatically cut the tissue once sealed or the instrument may be configured to permit the surgeon to selectively divide the tissue once sealed. Moreover, it is envisioned that an audible or visual indicator (not shown) may be triggered by a sensor (not shown) to alert the surgeon when an effective seal has been created. The sensor may, for example, determine if a seal is complete by measuring one of tissue impedance, tissue opaqueness and/or tissue temperature.

From the foregoing and with reference to the various figure drawings, those skilled in the art will appreciate that certain modifications can also be made to the present disclosure without departing from the scope of the present disclosure. For example, cutting element may be dimensioned as a cutting wire that is selectively activatable by the surgeon to divide the tissue after sealing. More particularly, a wire is mounted within the insulator between the jaw members and is selectively energizable upon activation of the switch.

The forceps may be designed such that it is fully or partially disposable depending upon a particular purpose or to achieve a particular result. For example, the electrode assembly may be selectively and releasably engageable with the distal end of the shaft and/or the proximal end of shaft may be selectively and releasably engageable with the housing and the handle assembly. In either of these two instances, the forceps would be considered "partially disposable" or "reposable", i.e., a new or different electrode assembly (or electrode assembly and shaft) selectively replaces the old electrode assembly as needed.

The electrode assembly could be selectively detachable (i.e., reposable) from the shaft depending upon a particular purpose, e.g., specific forceps could be configured for different tissue types or thicknesses. Moreover, a reusable forceps could be sold as a kit having different electrodes assemblies for different tissue types. The surgeon simply selects the appropriate electrode assembly for a particular tissue type.

The forceps could also include a mechanical or electrical lockout mechanism that prevents the sealing surfaces and/or the cutting element from being unintentionally activated when the jaw members are disposed in the open configuration.

Although the subject forceps and electrode assemblies have been described with respect to preferred embodiments, it will be readily apparent to those having ordinary skill in the art to which it appertains that changes and modifications may be made thereto without departing from the spirit or scope of the subject devices. For example, although the specification and drawing disclose that the electrically conductive surfaces may be employed to initially seal tissue prior to electrically cutting tissue in one of the many ways described herein, the electrically conductive surfaces may be configured and electrically designed to perform any known bipolar or monopolar function such as electrocautery, hemostasis, and/or desiccation utilizing one or both jaw members to treat the tissue. Moreover, the jaw members in their presently described and illustrated formation may be energized to simply cut tissue without initially sealing tissue which may prove beneficial during particular surgical procedures. Moreover, the various geometries of the jaw members, cutting elements, insulators and semi-conductive materials and the various electrical configurations associated therewith may be utilized for other surgical instrumentation depending upon a particular purpose, e.g., cutting instruments, coagulation instruments, electrosurgical scissors, etc.

## Claims

1. An electrode assembly for use with an instrument for sealing and cutting vessels and/or tissue, the electrode assembly comprising:
a pair of opposing first and second jaw members at least one of which being movable relative to the other from a first position wherein the jaw members are disposed in spaced relation relative to one another to a second position wherein the jaw members cooperate to grasp tissue therebetween;
each jaw member including an insulator and at least one electrically conductive tissue sealing surface extending along a length thereof, each tissue sealing surface being adapted to connect to a source of electrosurgical energy such that the at least one electrically conductive tissue sealing surfaces are capable of conducting electrosurgical energy through tissue held therebetween to effect a seal, the at least one electrically conductive tissue sealing surface having an exposed surface area;
at least one electrically conductive cutting element disposed within the insulator of the first jaw member, the electrically conductive cutting element disposed in general vertical registration to the insulator on the second jaw member defining at least one cutting zone between the at least one electrically conductive tissue sealing surfaces and the cutting element; and
an insulative material disposed between at least one of the electrically conductive tissue sealing surfaces of the first jaw member and the cutting element, the insulative material configured to reduce the exposed surface area of at least one electrically conductive tissue sealing surface of the first jaw member to optimize the power density for at least one of sealing and cutting tissue.

2. The electrode assembly according to claim 1, wherein the insulative material is selected from the group consisting of glass, ceramic and polymeric materials.

3. The electrode assembly according to any preceding claim, wherein the exposed surface area of each electrically conductive tissue sealing surface is substantially equivalent.

4. The electrode assembly according to any preceding claim, wherein the exposed area of one electrically conductive tissue sealing surface is greater than the exposed area of another electrically conductive tissue sealing surface.

5. The electrode assembly according to any preceding claim, wherein an insulative material is disposed upon the electrically conductive cutting element.

6. The electrode assembly according to any preceding claim, wherein the pair of opposing first and second jaw members maintain one polarity and the electrically conductive cutting element maintains a different polarity in order to effect tissue cutting.

7. The electrode assembly according to any preceding claim, wherein the first jaw member has a polarity, the second jaw member has an opposing polarity and the electrically conductive cutting element has a neutral polarity.

8. The electrode assembly according to any preceding claim wherein the electrosurgical parameters of the cutting element are automatically configured based upon a desired end tissue thickness at the seal.

9. The electrode assembly according to any preceding claim wherein an initial pulse is delivered to the cutting element which electrically communicates with a feedback sensor to regulate the electrosurgical intensity to effectively and consistently cut tissue with minimal effect on the tissue seal.
